Europäisches Patentamt

European Patent Office

Office européen des brevets

(19)

(11) Publication number: **0 112 621**
**B1**

(12) **EUROPEAN PATENT SPECIFICATION**

(45) Date of publication of patent specification: **12.08.87**

(51) Int. Cl.⁴: **A 61 K 35/78, A 23 L 1/20**

(21) Application number: **83306461.1**

(22) Date of filing: **25.10.83**

(54) Plant extracts and their therapeutic use.

(30) Priority: **17.12.82 JP 222287/82**

(43) Date of publication of application:
**04.07.84 Bulletin 84/27**

(45) Publication of the grant of the patent:
**12.08.87 Bulletin 87/33**

(84) Designated Contracting States:
**CH DE FR GB LI**

(56) References cited:
**FR-A-2 395 288**
**GB-A-2 039 204**

(73) Proprietor: **TOKIWA KANPO
PHARMACEUTICAL CO., LTD.
No. 10-8 Abikohigashi 2-chome Sumiyoshi-ku
Osaka-shi Osaka-fu (JP)**

(72) Inventor: **Iwamura, Junichi
621-1 Takaida
Kashiwara-shi Osaka-fu (JP)**

(74) Representative: **Perry, Robert Edward et al
GILL JENNINGS & EVERY 53-64 Chancery Lane
London WC2A 1HN (GB)**

The file contains technical information
submitted after the application was filed and
not included in this specification

Courier Press, Leamington Spa, England.

# Description

The present invention relates to the production of plant extracts which can be used in the prophylactic and therapeutic treatment of obesity, i.e. in dietotherapy.

Obesity is a major social problem. For example, as the cause of increased cholesterol levels in the serum and liver, obesity may accelerate arteriosclerosis and fatty liver, the onset of maturity diabetes, coronary sclerosis and degenerative joint disease. It is desirable to monitor the body weight of anyone who has a tendency to become fat, in order to allow prophylaxis and early treatment of obesity.

The most common and conventional treatment of obesity is starvation. However, this is painful and difficult to implement. More recently, so-called "fat-reducers" have been marketed, but most of these contain an ingredient, such as an enzyme inhibitor, sufficiently toxic that it may impair good health.

GB—A—2039204 discloses a foodstuff, for human consumption, including a material containing at least 35% soybean polysaccharides and which is substantially free of fats, monosaccharides and other water-soluble components. The product is of utility in treating digestive disorders, hypertension and high cholesterol levels. The product may be prepared by a variety of procedures which are conventional for removing water-soluble components fats and proteinaceous materials; one of the procedures which is disclosed comprises extracting untoasted defatted soybean meats with acidified isoelectric water, and re-extracting the material with aqueous alkali; the residue is the desired soybean product.

According to the present invention, a process for producing a plant-derived product comprises extracting defatted seeds of a leguminous plant, first with alkali and then with acid, to obtain an unextractable residue which is acid- and alkali-insoluble. Such material can be digestible and free of undesirable side-effects. Its utility is surprising, since liguminous plant seeds usually have high nutritive values.

The defatted seeds of leguminous plants used in the invention include seeds of plants belonging to *Leguminosae* which are used commonly for foods or drugs, and which are pre-defatted. Typical examples of these seeds are soybeans, black soybeans, broad beans, peas, red beans, peanuts, seeds of *Cassia tora* and seeds of *Cassia obtusifolia* (Cassia seed).

Defatting may be carried out in conventional manner. In the first step of a typical procedure, seeds are crushed into appropriate sizes (usually to a size 6—8 times smaller than before) and, if desired, separated from the seed coat. The crushed seeds are treated at 100—130 C, in order to coagulate protein, and then rolled using, for example, a corrugated roller mill. The rolled pieces are extracted with a non-polar organic solvent of which examples are petroleum-derived solvents such as petroleum ether or n-hexane. The extraction is usually carried out, with heating, at around 50 C. The extract is evaporated to recover oil.

In the process of the invention, alkali extraction precedes acid extraction. A dilute aqueous solution (normally containing not more than 1% of, for example, sodium hydroxide or potassium carbonate) can be used for the alkali extraction, by which water-soluble proteins are removed. The acid extraction can be carried out with a dilute aqueous solution (normally about pH 5) of a mineral or organic acid, preferably an organic acid such as acetic acid or propionic acid. In each case, the ambient temperature is suitable for extraction.

After the extraction, the residue is washed with water and dried. For drying, vacuum-drying or lyophilisation is preferred; air-drying may be used but the residue is then liable to become coloured.

The acid- and alkali-insoluble substance thus obtained comprises fibrous materials. These can be used as a health food, either as such or as part of a formulation commonly used as a medicine for internal administration, such as tablets, pills, granules, powders or capsules, prepared by powdering the substance and, if necessary, mixing it with excipient. Alternatively, the substance may be mixed into foods such as yoghurts, sweets, biscuits or jellies, or it may be mixed with, say, other pharmaceuticals or herb medicines.

Materials prepared according to the invention can prevent adiposity and inhibit increase in body weight. They therefore have utility in the prophylaxis and therapy of adult diseases such as arteriosclerosis, fatty liver, diabetes and rectal carcinoma, and the maintenance or recovery of health.

The following Example illustrates the invention.

Example

To 1 part of defatted soybean (extracting solvent n-hexane) were added 7—15 parts of 4% aqueous sodium hydroxide solution, and the mixture was stirred at room temperature for 1—1.5 hours. Solids were collected by filtration or centrifugation, and 7—15 parts of water were added thereto. The mixture was adjusted to pH 5 with acetic acid and stirred at room temperature for 10—20 minutes. Precipitates of proteins formed in the acidic medium were removed using a net having apertures between 0.42 mm and 1.41 mm in size (12 mesh and 36 mesh JIS), and the fibrous extraction residue was washed with water. Water was then removed by filtration under pressure or centrifugation, and the residue was dried at low temperature under reduced pressure to give a product (yield 26.3—38.2%).

Analysis of the product is as follows (total pectin was determined, by carbazole colorimetry, as galacturonic acid, and cellulose was determined by the method of P. J. Van Soest):

| | |
|---|---|
| Proteins (factor 5.71) | 31.4% |
| Fibres | 18.4% |
| Ash | 5.4% |
| Total pectin | 5.34% |
| Cellulose | 25.5% |
| Calcium | 1.24% |
| Potassium | 195 mg/100 g |
| Iron | 64.9 mg |

Defatted black soybeans or defatted peanuts could by treated in a similar manner.

In one example of a formulation, 150 parts of the product of the Example and 90 parts lactose were mixed and introduced into a hard-shell gelatin capsule.

In another example of a formulation, the following were mixed and baked into a biscuit:

| | |
|---|---|
| Product of the Example | . 80 parts |
| Wheat flour | 100 parts |
| Shortening | 15 parts |
| Sugar | 13 parts |
| Condensed milk | 6 parts |
| Refined sugar | 18 parts |
| Sodium bicarbonate | 0.6 part |
| Butter | 10 parts |
| Water | 6 parts |

Effects of the product prepared by the process of the invention, respectively on body weight and with respect to acute toxicity, have been determined in tests. In each case, a test liquid was prepared by suspending 20 g of the finely-divided product in distilled water, to make 100 ml of suspension. The test liquid was administered perorally once, by forces feeding through a stomach tube, to mice about 5 weeks old, strain dd Y-N. The tests were conducted at room temperature ($22 \pm 2$ C).

In the first test, male mice having an initial body weight of 20 g were used. They were weighed again 7 days after administration. At dosages of 6.9 g/kg, 8.3 g/kg, 10 g/kg and 12 g/kg, the mean weight gains of respective groups of 8 mice over 7 days were 6.5 g, 3.8 g, 5.0 g and 5.3 g. For control mice (nil dosage), the mean weight gain over 7 days was 7.5 g. Even at a single dosage of 6.9 g/kg, therefore, body weight increase was reduced.

In the second test, administration was to groups of 10 male (20—22 g initial body weight- or female (18—20 g initial body weight) mice. $LD_{50}$ was calculated by the probit method.

At dosages of 6.9 g/kg, 8.3 g/kg, 10 g/kg and 12 g/kg to four groups each of 10 male mice and also to four groups each of 10 female mice, no mortality was observed in any case after 5 hours, 15 hours, 1, 2, 3, 4, 5, 6 or 7 days. No disorder was observed in either male or female groups, immediately after administration, and growth was apparently normal afterwards. On autopsy, no abnormality in main internal organs was observed.

The dosage of 1200 mg/kg corresponds to an administered dose of 60 ml/kg which was considered to be the upper limit of one peroral administration of the test liquid to a mouse. The concentration of 20% was considered to be the maximun concentration which could be administered through a stomach tube, owing to the fact that the product swells when suspended in water. It was concluded that the peroral $LD_{50}$ of the product was more than 12000 mg/kg for both male and female groups, and that the product is of extremely low toxicity.

## Claims

1. A process for producing a plant-derived product, which comprises extracting defatted seeds of a leguminous plant, first with alkali and then with acid, to obtain an unextractable residue which is acid- and alkali-insoluble.

2. A process according claim 1, in which acid extraction is conducted using acetic acid or propionic acid.

## Patentansprüche

1. Verfahren zur Herstellung eines pflanzlichen Produkts, umfassend das Extrahieren entfetteter Samen einer Leguminosen-Pflanze zunächst mit Alkali und dann mit Säure, wonach ein unextrahierbarer Rückstand erhalten wird, der in Säure und Alkali unlöslich ist.

2. Verfahren nach Anspruch 1, dadurch gekennzeichnet, daß die Säure-Extraktion unter Verwendung von Essigsäure oder Propionsäure durchgeführt wird.

## Revendications

1. Procédé pour la préparation d'un produit dérivé de plantes qui comprend l'extraction de graines dégraissées d'une plante légumineuse, tout d'abord avec un agent alcalin puis ensuite avec un acide, de façon à obtenir un résidu non extractible qui est insoluble en milieu acide et alcalin.

2. Procédé selon la revendication 1 dans lequel l'extraction est réalisée en utilisant de l'acide acétique ou propionique.